# EUROPEAN PATENT SPECIFICATION

(11) **EP 0 617 620 B1**
(45) Date of publication and mention of the grant of the patent: **09.09.1998**
(21) Application number: 93900327.3
(22) Date of filing: 21.12.1992
(51) Int. Cl.: A61K 31/40, A61K 31/445, A61K 31/435

(54) **USE OF 5-HT4 - ANTAGONISTS FOR THE MANUFACTURE OF A MEDICAMENT FOR THE TREATMENT OF URINARY INCONTINENCE**
VERWENDUNG VON 5-HT4 - ANTAGONISTEN ZUR HERSTELLUNG EINES ARZNEIMITTELS ZUR BEHANDLUNG VON HARNINKONTINENZ
UTILISATION D'ANTAGONISTES AU RECEPTEUR 5-HT 4 POUR FABRIQUER UN MEDICAMENT POUR LE TRAITEMENT DE L'INCONTINENCE URINAIRE

(30) Priority: 21.12.1991 GB 9127184; 21.12.1991 GB 9127185; 12.09.1992 GB 9219354
(43) Date of publication of application: 05.10.1994
(73) Proprietor: SMITHKLINE BEECHAM PLC, Brentford, Middlesex TW8 9EP (GB)
(72) Inventor: SANGER, Gareth John SmithKline Beecham, Harlow Essex CM19 5AD (GB); WARDLE, Kay Alison SmithKline Beecham, Harlow Essex CM19 5AD (GB)
(74) Representative: Tocher, Pauline
(86) International application number: GB9202376
(87) International publication number: WO9312785

(56) References cited:
- EP-A- 0 467 365
- EP-A- 0 501 322
- PARAPLEGIA vol. 26, no. 3, 1988, pages 162 - 164; M.ETIENNE ET AL.: 'Treatment with cisapride of the gastrointestinal and urological sequelae of spinal cord transection: case report'
- PARAPLEGIA vol. 26, no. 3, 1988, pages 159 - 161; G.H. DE GROOT ET AL.: 'Effects of cisapride on constipation due to neurological lesion'
- ACTA BELG. MED. PHYS. vol. 12, no. 3, 1989, pages 81 - 88; P.HANSON ET AL.: 'Effet du cisapride sur les vessies neurologiques'
- DRUG. DEV. RES. vol. 27, no. 4, 1992, pages 361 - 375; WILLIAM D. STEERS ET AL.: 'Effects of serotonic agonists on micturation and sexual function in the rat'
- DRUGS FUTURE vol. 16, no. 11, 1991, pages 1011 - 1026; M. TURCONI ET AL: 'Azabicycloalkyl benzimidazolones: Interaction with serotonergic 5-HT3 and 5-HT4 receptors and potential therapeutic implications'

## Description

This invention relates to treatment of urinary incontinence.

European Journal of Pharmacology 146 (1988), 187-188, and Naunyn-Schmiedeberg's Arch. Pharmacol. (1989) 340:403-410, describe a non classical 5-hydroxytryptamine receptor, now designated the 5-HT₄ receptor, and that tropisetron (ICS 205-930), which is also a 5-HT₃ receptor antagonist, acts as an antagonist at this receptor and metoclopramide is an agonist at this receptor.

WO 91/16045 (SmithKline and French Laboratories Limited) describes the use of cardiac 5-HT₄ receptor antagonists in the treatment of atrial arrhythmias and stroke.

Metoclopramide has been shown to be effective in treating a poorly functioning bladder, (Scand. J. Urology and Nephrology, 13:79-82 (1979) but this has not been specifically linked to any known action of metoclopramide.

There are reports in the literature of 5-HT₄ receptors potentiating contractions in human bladder (Br. J. Pharmacol, 61, 115P) and inhibiting contractions in monkey bladder (2nd International Symposium on Serotonin, Houston, September 1992, page 86).

Paraplegia, vol. 26, no. 3, 1988, pages 159-161, Paraplegia, vol. 26, no. 3, 1988, pages 162-164, and Acta Belg. Med. Phys., vol. 12, no. 3, 1989, pages 81-88, describe the effects of cisapride on bladder function. EP-A-467365 (E. R. Squibb & Sons, Inc.) relates to the use of 5-HT₃ receptor antagonists, including zacopride, which is also a 5-HT₄ receptor agonist, in the treatment of urinary incontinence.

We have now discovered that a compound which acts as an antagonist at 5-HT₄ receptors is of potential use in the treatment of urinary incontinence, which is often associated with irritable bowel syndrome (IBS).

The invention therefore provides the use of a 5-HT₄ receptor antagonist in the manufacture of a medicament for use in the treatment and/or prophylaxis of urinary incontinence.

5-HT₄ receptor antagonists may be identified according to standard methods, such as those described hereinafter, and that described in Naunyn-Schmiedeberg's Arch Pharmacol. 342, 619-622.

Examples of 5-HT₄ receptor antagonists include ICS 205-930 (tropisetron - Sandoz), R 50 595 (Janssen), which is described in FR 76530 and Eur.J. Pharmacol., 181 119-125 (1990), and SDZ 205-557, which is described by K.H. Buchheit and R. Gamse in Naunyn-Schmiedeberg's Arch. Pharmacol., 343 (Suppl.), R101 (1991). DAU 6285 (Naunyn-Schmiedeberg's Arch. Pharmacol, 345; 264-269 (1992) and RS 23597-190 (Syntex - British Pharmacology Society Meeting, September 1992).

EP-A-501322 (Glaxo Group Limited) describes indole derivatives having 5-HT₄ receptor antagonist activity of formula: 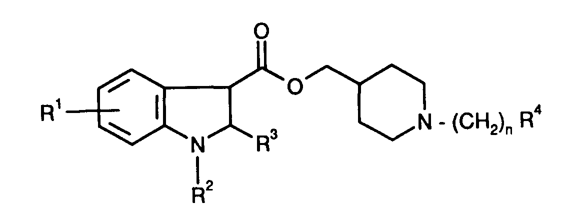
wherein R¹ represents a hydrogen or a halogen atom, or a C₁₋₆ alkyl, C₁₋₆ alkoxy or hydroxy group;
R² represents a hydrogen atom or a C₁₋₆ alkyl, -CH₂C₂₋₅ alkenyl or -CH₂C₂₋₅ alkynyl group;
R³ represents a hydrogen atom or a C₁₋₆ alkyl or C₁₋₆ alkoxy group;
n represents 2 or 3;
R⁴ represents a group selected from cyano, hydroxyl, C₁₋₆ alkoxy, phenoxy, C(0)C₁₋₆ alkyl, C(0)C₆H₅, -CONR⁵R⁶, -NR⁵COR⁶, -SO₂NR⁵R⁶ or -NR⁵SO₂R⁶ (wherein each of R⁵ and R⁶ independently represent a hydrogen atom, a C₁₋₆ alkyl or phenyl group);
and quaternary ammonium derivatives, piperidine N-oxides and pharmaceutically acceptable salts and solvates thereof;
and reports 5-HT₄ receptors are believed to be associated with conditions involving *inter alia* the urinary tract (e.g. urinary incontinence).

In one aspect, the 5-HT₄ receptor antagonist is more potent at 5-HT₄ receptors than at 5-HT₃ receptors.

Preferably, the 5-HT₄ receptor antagonist is in substantially pure pharmaceutically acceptable form.

The administration of the 5-HT₄ receptor antagonist may be by way of oral, sublingual, transdermal or parenteral administration.

An amount effective to treat the disorder hereinbefore described depends on the usual factors such as the nature and severity of the disorder being treated and the weight of the mammal. However, a unit dose will normally contain 0.1 to 50 mg for example 0.5 to 10 mg, of the 5-HT₄ receptor antagonist. Unit doses will normally be administered once or more than once a day, for example 2, 3, or 4 times a day, more usually 1 to 3 times a day, such that the total daily dose is normally in the range, for a 70 kg adult of 0.1 to 50 mg, for example 0.1 to 5 mg, that is in the range of approximately 0.001 to 1 mg/kg/day, more usually 0.005 to 0.2 mg/kg/day.

For oral or parenteral administration, it is greatly preferred that the 5-HT₄ receptor antagonist is administered in the form of a unit-dose composition, such as a unit dose oral or parenteral composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling or tabletting. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl *p*-hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing the 5-HT₄ receptor antagonist and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

As is common practice, the compositions will usually be accompanied by written or printed directions for use in the treatment concerned.

### 5-HT₄ receptor antagonist activity

### 1) Guinea pig colon

Male guinea-pigs, weighing 250-400g are used. Longitudinal muscle-myenteric plexus preparations, approximately 3cm long, are obtained from the distal colon region. These are suspended under a 0.5g load in isolated tissue baths containing Krebs solution bubbled with 5% CO₂ in O₂ and maintained at 37°C. In all experiments, the Krebs solution also contains methiothepin 10⁻⁷Mn and granisetron 10⁻⁶M to block effects at 5-HT₁, 5-HT₂ and 5-HT₃ receptors.

After construction of a simple concentration-response curve with 5-HT, using 30s contact times and a 15min dosing cycle, a concentration of 5-HT is selected so as to obtain a contraction of the muscle approximately 40-70% maximum(10-9M approx). The tissue is then alternately dosed every 15min with this concentration of 5-HT and then with an approximately equi-effective concentration of the nicotine receptor stimulant, dimethylphenylpiperazinium (DMPP). After obtaining consistent responses to both 5-HT and DMPP, increasing concentrations of a putative 5-HT₄ receptor antagonist are then added to the bathing solution. The effects of this compound are then determined as a percentage reduction of the contractions evoked by 5-HT or by DMPP.

From this data, IC₅₀ values are determined, being defined as the concentration of antagonist which reduces or increases the contraction by 50%. A compound which reduces the response to 5-HT but not to DMPP is believed to act as a 5-HT₄ receptor antagonist.

### 2) Rat oesophagus

Rat oesophageal tunica muscularis mucosae is set up according to Baxter *et. al.* Naunyn-Schmiedeberg's Arch. Pharmacol., 343, 439-446 (1991). The inner smooth muscle tube of the muscularis mucosae is isolated and mounted for isometric tension recording in oxygenated (95% O₂/5% CO₂) Tyrodes solution at 37°C. All experiments are performed in pargyline pre-treated preparations (100µM for 15 min followed by washout) and in the presence of cocaine (30µM). Relaxant responses to 5-HT are obtained after pre-contracting the oesophagus tissue with carbachol (3µM).

## Claims

1. The use of a 5-HT₄ receptor antagonist in the manufacture of a medicament for use in the treatment and/or prophylaxis of urinary incontinence.

2. A use according to claim 1 for the treatment of urinary incontinence associated with irritable bowel syndrome.

3. A use according to claim 1 or 2 wherein 5-HT₄ receptor antagonist is R 50 595, SDZ 205-557, DAU 6285, RS 23597-190 or a compound described in relation to EP-A-501322 (Glaxo Group Limited) of formula: 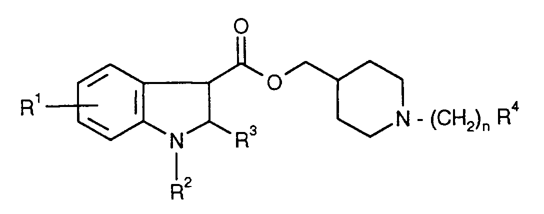
wherein R¹ represents a hydrogen or a halogen atom, or a C₁₋₆ alkyl, C₁₋₆ alkoxy or hydroxy group;
R² represents a hydrogen atom or a C₁₋₆ alkyl, -CH₂C₂₋₅ alkenyl or -CH₂C₂₋₅ alkynyl group;
R³ represents a hydrogen atom or a C₁₋₆ alkyl or C₁₋₆ alkoxy group;
n represents 2 or 3;
R⁴ represents a group selected from cyano, hydroxyl, C₁₋₆ alkoxy, phenoxy, C(0)C₁₋₆ alkyl, C(0)C₆H₅, -CONR⁵R⁶, -NR⁵COR⁶, -SO₂NR⁵R⁶ or -NR⁵SO₂R⁶ (wherein each of R⁵ and R⁶ independently represent a hydrogen atom, a C₁₋₆ alkyl or phenyl group);
and quaternary ammonium derivatives, piperidine N-oxides and pharmaceutically acceptable salts and solvates thereof.

4. A use according to any one of claims 1 to 3 wherein the 5-HT₄ receptor antagonist is more potent at 5-HT₄ receptors than 5-HT₃ receptors.

## Patentansprüche

1. Verwendung eines 5-HT₄-Rezeptor-Antagonisten zur Herstellung eines Medikaments zur Verwendung bei der Behandlung und/oder Prophylaxe von Harninkontinenz.

2. Verwendung nach Anspruch 1 zur Behandlung von Harninkontinenz, die mit dem Reizkolon-Syndrom verbunden ist.

3. Verwendung nach Anspruch 1 oder 2, wobei der 5-HT₄-Rezeptor-Antagonist R 50 595, SDZ 205-557, DAU 6285, RS 23597-190 oder eine unter Bezug auf EP-A-501322 (Glaxo Group Limited) beschriebene Verbindung der Formel: 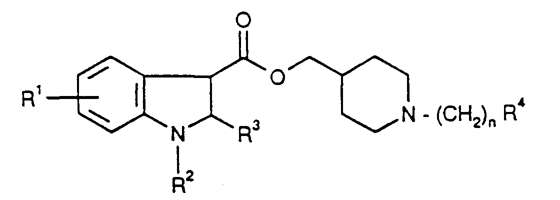 ist, in der
R¹ ein Wasserstoff- oder ein Halogenatom oder einen C₁₋₆-Alkyl-, C₁₋₆-Alkoxy- oder Hydroxyrest bedeutet;
R² ein Wasserstoffatom oder einen C₁₋₆-Alkyl-, ―CH₂C₂₋₅-Alkenyl- oder ―CH₂C₂₋₅-Alkinylrest bedeutet;
R³ ein Wasserstoffatom oder einen C₁₋₆-Alkyl- oder C₁₋₆-Alkoxyrest bedeutet;
n 2 oder 3 bedeutet;
R⁴ einen Rest, ausgewählt aus Cyano, Hydroxyl, C₁₋₆-Alkoxy, Phenoxy, C(O)C₁₋₆-Alkyl, C(O)C₆H₅, ―CONR⁵R⁶, ―NR⁵COR⁶, ―SO₂NR⁵R⁶ oder ―NR⁵SO₂R⁶ darstellt (wobei jeder der Reste R⁵ und R⁶ unabhängig voneinander ein Wasserstoffatom, einen
C₁₋₆-Alkylrest oder eine Phenylgruppe bedeutet);
und quaternäre Ammoniumderivate, Piperidin-N-oxide und pharmazeutisch verträgliche Salze und Solvate davon.

4. Verwendung nach einem der Ansprüche 1 bis 3, wobei der 5-HT₄-Rezeptor-Antagonist stärker an 5-HT₄-Rezeptoren als an 5-HT₃-Rezeptoren wirkt.

## Revendications

1. Utilisation d'un antagoniste du récepteur 5-HT₄ dans la production d'un médicament destiné à être utilisé dans le traitement et/ou la prophylaxie de l'incontinence urinaire.

2. Utilisation suivant la revendication 1 pour le traitement de l'incontinence urinaire associée au syndrome du côlon irritable.

3. Utilisation suivant la revendication 1 ou 2, dans laquelle l'antagoniste du récepteur 5-HT₄ est le R 50 595, le SDZ 205-557, le DAU 6285, le RS 23597-190 ou un composé décrit en rapport avec le document EP-A-501 322 (Glaxo Group Limited) de formule : 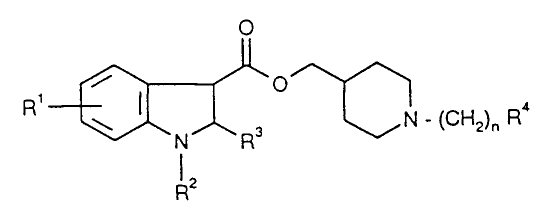
dans laquelle R¹ représente un atome d'hydrogène ou un atome d'halogène, ou bien un groupe alkyle en C₁ à C₆, alkoxy en C₁ à C₆ ou hydroxy ;
R² représente un atome d'hydrogène ou un groupe alkyle en C₁ à C₆, -CH₂ (alcényle en C₂ à C₅) ou -CH₂ (alcynyle en C₂ à C₅) ;
R³ représente un atome d'hydrogène ou un groupe alkyle en C₁ à C₆ ou alkoxy en C₁ à C₆ ;
n est égal à 2 ou 3 ;
R⁴ représente un groupe choisi entre des groupes cyano, hydroxyle, alkoxy en C₁ à C₆, phénoxy, C(0)(alkyle en C₁ à C₆), C(0)C₆H₅, -CONR⁵R⁶, -NR⁵COR⁶, -SO₂NR⁵R⁶ et -NR⁵SO₂R⁶ (dans lesquels chacun des groupes R⁵ et R⁶ représente, indépendamment, un atome d'hydrogène, un groupe alkyle en C₁ à C₆ ou un groupe phényle) ;
ou un de ses dérivés d'ammonium quaternaire, pipéridine-N-oxydes et sels et produits de solvatation pharmaceutiquement acceptables.

4. Utilisation suivant l'une quelconque des revendications 1 à 3, dans laquelle l'antagoniste du récepteur 5-HT₄ est plus actif au niveau des récepteurs 5-HT₄ qu'au niveau des récepteurs 5-HT₃.
